# EUROPEAN PATENT APPLICATION

(11) **EP 4 458 386 A1**
(43) Date of publication of application: **06.11.2024**
(21) Application number: 23749660.9
(22) Date of filing: 26.01.2023
(51) Int. Cl.: A61M 5/168

(54) **INFUSION PUMP**

(30) Priority: 02.02.2022 JP 2022015247
(71) Applicant: Terumo Kabushiki Kaisha, Tokyo 151-0072 (JP)
(72) Inventor: IDE, Jun, Ashigarakami-gun, Kanagawa 259-0151 (JP); HONDA, Osamu, Ashigarakami-gun, Kanagawa 259-0151 (JP); HARADA, Katsunori, Tokyo 106-6135 (JP)
(74) Representative: TBK
(86) International application number: PCT/JP2023/002459
(87) International publication number: WO 2023/149344

(57) **Abstract**

An infusion pump according to the present disclosure includes a displacement sensor including a groove portion to which an infusion tube is attached and a detection surface exposed to the groove portion, and detecting a pressure applied to the detection surface, in which the displacement sensor includes a first mask portion that covers one end of the detection surface in a groove width direction of the groove portion, and a second mask portion that covers the other end of the detection surface in the groove width direction of the groove portion.

## Description

### Technical Field

The present disclosure relates to an infusion pump.

### Background Art

Conventionally, a technique is known for detecting an abnormality of an infusion tube attached to an infusion pump, in the infusion pump for delivering a drug or the like to a patient. For example, in Patent Literature 1, an infusion pump is disclosed that includes an occlusion sensor capable of detecting an occlusion state of an infusion tube.

### Citation List

### Patent Literature

Patent Literature 1: JP 2012-200545 A

### Summary of Invention

### Technical Problem

However, in the technique for detecting an abnormality of an infusion tube attached to an infusion pump, further improvement of usefulness thereof is still required. For example, it is required to detect whether or not the infusion tube is correctly attached to the infusion pump.

An object of the present disclosure made in view of such circumstances is to provide an infusion pump that improves the usefulness of the technique for detecting an abnormality of an infusion tube attached to an infusion pump.

### Solution to Problem

An infusion pump according to an embodiment of the present disclosure includes a displacement sensor including a groove portion to which an infusion tube is attached and a detection surface exposed to the groove portion, and detecting a pressure applied to the detection surface, in which the displacement sensor includes a first mask portion that covers one end of the detection surface in a groove width direction of the groove portion, and a second mask portion that covers the other end of the detection surface in the groove width direction of the groove portion.

In the infusion pump according to the embodiment of the present disclosure, it is preferable that the displacement sensor includes a Hall element and a plunger movable with respect to the Hall element and including a magnet, and the displacement sensor is configured to detect a pressure applied to the detection surface on the basis of an amount of movement of the magnet with respect to the Hall element.

In the infusion pump according to the embodiment of the present disclosure, it is preferable that the groove portion, the first mask portion, and the second mask portion are provided in a mask member, and the mask member is detachable from the displacement sensor.

In the infusion pump according to the embodiment of the present disclosure, it is preferable that a distance between the first mask portion and the second mask portion in the groove width direction of the groove portion is equal to a groove width of the groove portion.

In the infusion pump according to the embodiment of the present disclosure, it is preferable that a liquid delivery drive part is included capable of delivering liquid in the infusion tube, and the displacement sensor is provided on each of both sides of the liquid delivery drive part in a liquid delivery direction of the liquid delivery drive part.

It is preferable that the infusion pump according to the embodiment of the present disclosure includes: a main body unit provided with the displacement sensor; and a door unit that is openable and closable with respect to the main body unit and covers the displacement sensor in a state of being closed with respect to the main body unit, in which the door unit includes: a pressing member including a pressing surface facing the displacement sensor in a state where the door unit is closed with respect to the main body unit; and an urging member that urges the pressing member to cause the pressing surface of the pressing member to press the infusion tube against the displacement sensor in a state where the door unit is closed with respect to the main body unit.

In the infusion pump according to the embodiment of the present disclosure, it is preferable that the pressing surface of the pressing member includes a protruding portion, and the protruding portion is inserted into the groove portion from between the first mask portion and the second mask portion of the displacement sensor in a state where the door unit is closed with respect to the main body unit.

It is preferable that the infusion pump according to the embodiment of the present disclosure includes a notification unit, in which the notification unit outputs a warning in a case where erroneous attachment of the infusion tube is detected on the basis of the pressure detected by the displacement sensor.

In the infusion pump according to the embodiment of the present disclosure, it is preferable that the notification unit outputs a warning in a case where occlusion of the infusion tube is detected on the basis of the pressure detected by the displacement sensor.

In the infusion pump according to the embodiment of the present disclosure, it is preferable that the infusion pump is configured to be able to hold the infusion tube substantially horizontally.

### Advantageous Effects of Invention

According to the present disclosure, it is possible to provide an infusion pump that improves the usefulness of the technique for detecting an abnormality of an infusion tube attached to an infusion pump.

### Brief Description of Drawings

Fig. 1 is a diagram illustrating an infusion line including an infusion pump as an embodiment of the present disclosure.
Fig. 2 is a perspective view of the infusion pump illustrated in Fig. 1.
Fig. 3 is a front view of a state where a door unit of the infusion pump illustrated in Fig. 1 is closed.
Fig. 4 is a front view of a state where the door unit of the infusion pump illustrated in Fig. 1 is opened.
Fig. 5 is an exploded perspective view illustrating an example of a structure of a displacement sensor of the infusion pump illustrated in Fig. 4.
Fig. 6 is a perspective view of a mask member of the displacement sensor illustrated in Fig. 5.
Fig. 7 is a cross-sectional view of the mask member taken along a line I-I' in Fig. 6.
Fig. 8 is a perspective view illustrating an example of a structure of a pressing member of the infusion pump illustrated in Fig. 4.
Fig. 9 is a block diagram of the infusion pump illustrated in Fig. 1.

### Description of Embodiments

Hereinafter, an infusion pump according to an embodiment of the present disclosure will be described with reference to the drawings. In the drawings, common members and parts are denoted by the same reference signs. In the description of the present embodiment, the description of the common members and parts will be omitted or simplified as appropriate.

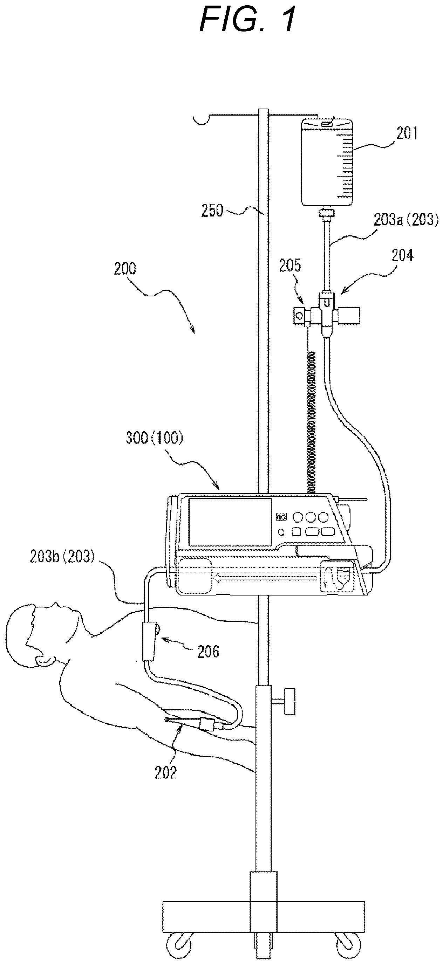

Fig. 1 is a diagram illustrating an infusion line 200 formed by connecting an infusion container 201 containing a liquid such as a drug solution to an indwelling needle 202 indwelled in a state of puncturing a patient with a tube 203 as an infusion tube. Hereinafter, the infusion container 201 side of the infusion line 200 may be referred to as a "flow path upstream side". In addition, the indwelling needle 202 side of the infusion line 200 may be referred to as a "flow path downstream side". Further, hereinafter, a direction from the flow path upstream side to the flow path downstream side of the infusion line 200 may be referred to as a liquid delivery direction A.

More specifically, the infusion line 200 illustrated in Fig. 1 includes an infusion container 201 hung on a stand 250, a drip tube 204, an indwelling needle 202, and two tubes 203. The two tubes 203 of the infusion line 200 are a first tube 203a connecting the infusion container 201 to the drip tube 204, and a second tube 203b connecting the drip tube 204 to the indwelling needle 202. In Fig. 1, a drip probe as a number-of-drops abnormality detection apparatus 205 is attached to the drip tube 204 of the infusion line 200. In addition, in Fig. 1, an infusion pump 300 as a liquid delivery pump is attached to the second tube 203b of the infusion line 200. Although details will be described later, the infusion pump 300 includes a displacement sensor 100 in order to detect an abnormality of the second tube 203b attached to the infusion pump 300. In addition, the infusion pump 300 is fixed to the stand 250. Further, a clamp 206 is attached to the second tube 203b of the infusion line 200 illustrated in Fig. 1 on the flow path downstream side from the infusion pump 300. In the following description, in a case where the first tube 203a and the second tube 203b are not particularly distinguished from each other, the tubes are simply collectively referred to as the tubes 203.

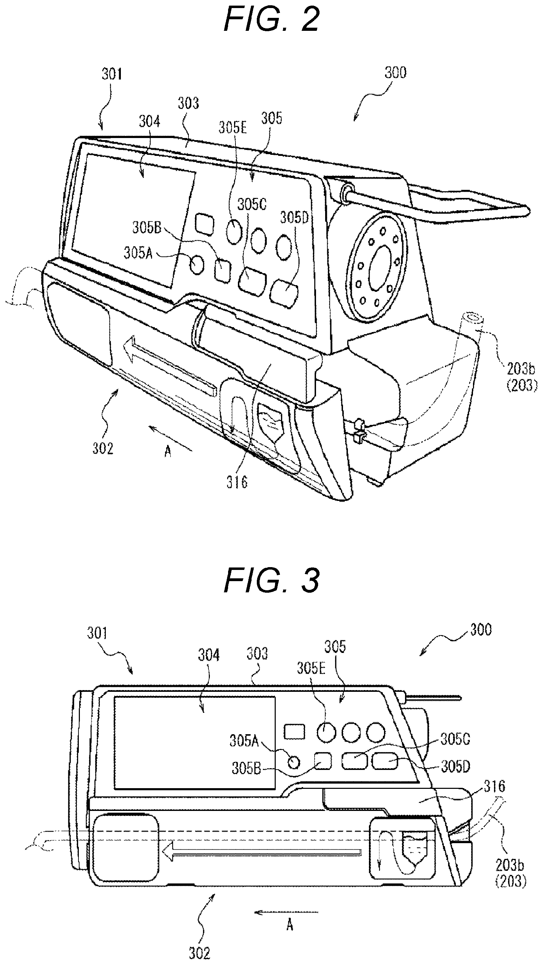

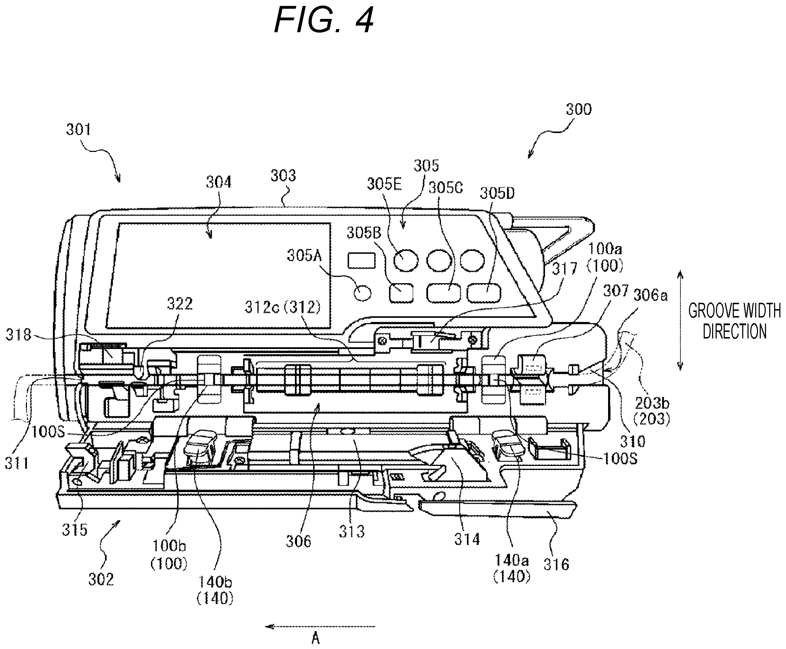

First, a schematic configuration will be described of the infusion pump 300 with reference to Figs. 2, 3, and 4. Fig. 2 is a perspective view of the infusion pump 300. Fig. 3 is a front view of a state where a door unit 302 of the infusion pump 300 is closed with respect to a main body unit 301. Fig. 4 is a front view of a state where the door unit 302 of the infusion pump 300 is opened with respect to the main body unit 301. In Figs. 2 to 4, the second tube 203b of Fig. 1 held by the infusion pump 300 is indicated by a two-dot chain line for convenience of description.

The infusion pump 300 illustrated in Figs. 2 to 4 is a liquid delivery pump used, for example, in an intensive care unit (ICU, CCU, NICU) or the like, and used to inject a liquid such as an anticancer agent, an anesthetic, a chemotherapeutic agent, a blood transfusion, or a nutrient for a patient for a relatively long time with an accuracy within ±10% (preferably an accuracy within ±3%) in a range of about 1 to 1200 mL/h (amount of injection per hour, that is, injection rate), 1 to 9999 mL (scheduled amount of injection).

The infusion pump 300 includes the main body unit 301 and the door unit 302 that can be opened and closed with respect to the main body unit 301. A main body cover 303 that is an exterior cover of the main body unit 301, and the door unit 302 described above are molded using a molding resin material having chemical resistance.

First, elements will be described arranged in the main body cover 303 of the main body unit 301 of the infusion pump 300. As illustrated in Figs. 2 to 4, a display unit 304 and an operation panel unit 305 are arranged on a front portion of the main body cover 303. The display unit 304 is an image display apparatus such as a color liquid crystal display apparatus. The display unit 304 is located on the left side of a front upper portion of the main body cover 303 and is arranged above the door unit 302.

On the display unit 304, a scheduled amount of injection (mL) of administration, an integrated amount (mL) of administration, a charge history, an injection rate (mL/h), and the like are displayed. In addition, a warning message may be displayed on the display unit 304.

The operation panel unit 305 is arranged on the right side of the front upper portion of the main body cover 303. In the operation panel unit 305, for example, a power switch button 305A, a fast delivery switch button 305B, a start switch button 305C, a stop switch button 305D, and a menu selection button 305E are arranged as operation buttons.

As illustrated in Figs. 2 to 4, the door unit 302 is attached to a front lower portion of the main body cover 303 so as to be rotatable about a rotation axis. The door unit 302 is a plate-like lid extending long along the left-right direction in a front view of the infusion pump 300 in Figs. 3 and 4, and can sandwich and hold the second tube 203b made of a thermoplastic resin such as soft vinyl chloride, for example, between the main body unit 301 and the door unit 302 in a state where the door unit 302 is closed with respect to the main body unit 301 (see Figs. 2 and 3). Thus, the infusion pump 300 is configured to be able to hold the second tube 203b substantially horizontally along the left-right direction in the front view (see Figs. 3 and 4).

In the present disclosure, "substantially horizontal" preferably means horizontal, but may include being inclined within a range of a predetermined angle with respect to a horizontal direction (a direction perpendicular to a direction in which gravity acts). The range of the predetermined angle is, for example, a range between -10 degrees and 10 degrees, but is not limited thereto.

As illustrated in Fig. 4, the second tube 203b is attached to a tube attachment unit 306 of the main body unit 301 exposed to the outside in a state where the door unit 302 of the infusion pump 300 is opened. As illustrated in Fig. 4, the tube attachment unit 306 is provided along the left-right direction below the display unit 304 and the operation panel unit 305 in the front view of the infusion pump 300. As illustrated in Figs. 2 and 3, when the door unit 302 is closed with respect to the main body unit 301, the tube attachment unit 306 is covered by the door unit 302.

As illustrated in Fig. 4, the tube attachment unit 306 defines a guide groove 306a to which the second tube 203b is attached. In the present embodiment, the guide groove 306a is continuously provided in the tube attachment unit 306 so as to extend in the left-right direction in the front view of the infusion pump 300, but may be intermittently provided. The second tube 203b is attached to the tube attachment unit 306 along the guide groove 306a. The tube attachment unit 306 includes an air bubble detection sensor 307, an upstream-side displacement sensor 100a, a downstream-side displacement sensor 100b, a first tube guide part 310, a second tube guide part 311, a liquid delivery drive part 312, and a tube clamp part 322. More specifically, in the tube attachment unit 306, the first tube guide part 310, the air bubble detection sensor 307, the upstream-side displacement sensor 100a, the liquid delivery drive part 312, the downstream-side displacement sensor 100b, the tube clamp part 322, and the second tube guide part 311 are arranged in this order from the upstream side in the liquid delivery direction A along the guide groove 306a. Inside the door unit 302, a tube pressing member 313 and engaging members 314 and 315 are provided.

The tube pressing member 313 is arranged as a long rectangular planar protruding portion along the left-right direction of the infusion pump 300 in the front view (see Fig. 4 and the like), and is at a position facing the liquid delivery drive part 312. The engaging members 314 and 315 are respectively fitted into fitting portions 317 and 318 formed in the main body unit 301 by operation of a lever 316 interlocked with the engaging members 314 and 315. As a result, the door unit 302 can be closed and the tube attachment unit 306 of the main body unit 301 can be closed. The tube clamp part 322 does not occlude the second tube 203b attached to the tube attachment unit 306 in a state where the door unit 302 is closed with respect to the main body unit 301. Then, the tube clamp part 322 clamps the second tube 203b attached to the tube attachment unit 306 and occludes a hollow portion of the second tube 203b in a state where the door unit 302 is opened with respect to the main body unit 301.

As illustrated in Fig. 4, the first tube guide part 310 is provided at the right end portion of the guide groove 306a of the tube attachment unit 306 in the front view of the infusion pump 300, and the second tube guide part 311 is provided at the left end portion of the guide groove 306a of the tube attachment unit 306 in the front view of the infusion pump 300. Then, the first tube guide part 310 can hold the second tube 203b by fitting the flow path upstream side of the second tube 203b of the infusion line 200 (see Fig. 1), and the second tube guide part 311 can hold the second tube 203b by fitting the flow path downstream side of the second tube 203b of the infusion line 200. That is, the infusion pump 300 can hold the second tube 203b substantially horizontally along the guide groove 306a by the first tube guide part 310 and the second tube guide part 311. As described above, the second tube 203b attached substantially horizontally is arranged so as to face the air bubble detection sensor 307, the upstream-side displacement sensor 100a, the liquid delivery drive part 312, the downstream-side displacement sensor 100b, and the tube clamp part 322 in order from the right in the front view of Fig. 4.

The air bubble detection sensor 307 is an apparatus that detects a bubble (air) contained in the liquid flowing in the second tube 203b.

The air bubble detection sensor 307 includes a transmitter and a receiver facing each other across the guide groove 306a. In the present embodiment, the transmitter and the receiver of the air bubble detection sensor 307 face each other in a groove width direction of the guide groove 306a. The air bubble detection sensor 307 transmits a signal from the transmitter toward the second tube 203b accommodated in the guide groove 306a, and applies the signal to the liquid flowing in the second tube 203b from the outside of the second tube 203b made of soft vinyl chloride, for example. At this time, since a transmittance of the signal in the liquid is different from a transmittance of the signal in the bubble, even in a case where the same signals are transmitted from the transmitter, a difference occurs between the signals received by the receiver depending on whether the bubble is included in the liquid flowing through the second tube 203b. The signal transmitted from the transmitter is, for example, an ultrasonic wave, but is not limited thereto. As described above, the air bubble detection sensor 307 can detect the bubble contained in the liquid flowing in the second tube 203b on the basis of the difference between the signals received by the receiver. The air bubble detection sensor 307 is communicably connected to an acquisition unit 351 described later by wired communication or wireless communication. As a result, the air bubble detection sensor 307 can transmit, for example, information such as a signal received by the receiver to the acquisition unit 351 as bubble detection information.

The upstream-side displacement sensor 100a and the downstream-side displacement sensor 100b are apparatuses that detect an abnormality of the infusion tube 203 (the second tube 203b in the present embodiment) attached to the infusion pump 300. In the present embodiment, the upstream-side displacement sensor 100a and the downstream-side displacement sensor 100b are made to have the same configuration. In the following description, in a case where the upstream-side displacement sensor 100a and the downstream-side displacement sensor 100b are not distinguished from each other, the displacement sensors are simply collectively referred to as the displacement sensors 100.

As illustrated in Fig. 4, the upstream-side displacement sensor 100a is arranged on the upstream side of the liquid delivery drive part 312 in the guide groove 306a of the tube attachment unit 306. Then, the downstream-side displacement sensor 100b is arranged on the downstream side from the liquid delivery drive part 312 in the guide groove 306a of the tube attachment unit 306. That is, the displacement sensors 100 are respectively provided on both sides of the liquid delivery drive part 312 in the liquid delivery direction A of the liquid delivery drive part 312. Here, the liquid delivery direction A of the liquid delivery drive part 312 is also an extending direction of the second tube 203b attached to the liquid delivery drive part 312. As a result, the displacement sensors 100 are respectively arranged on the upstream side and the downstream side of a portion of the second tube 203b to which force is applied from the liquid delivery drive part 312 for liquid delivery, whereby an abnormality of the second tube 203b is easily detected such as erroneous attachment or occlusion. However, the number of the displacement sensors 100 included in the tube attachment unit 306 may be set to any number greater than or equal to one. For example, the tube attachment unit 306 may be made to have a configuration in which only one displacement sensor 100 is included upstream or downstream of the liquid delivery drive part 312.

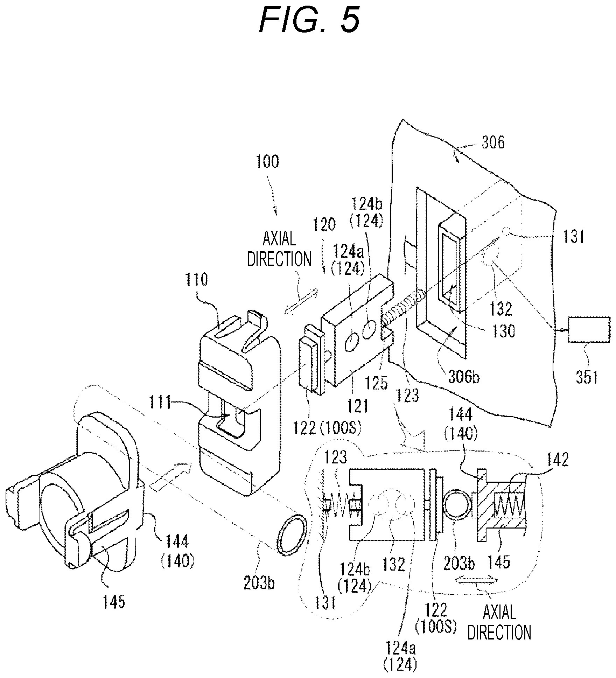

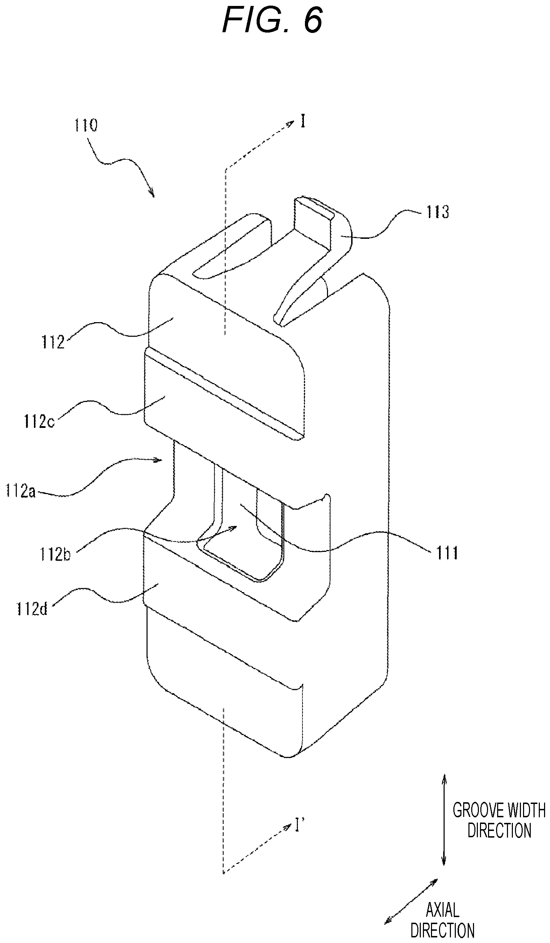

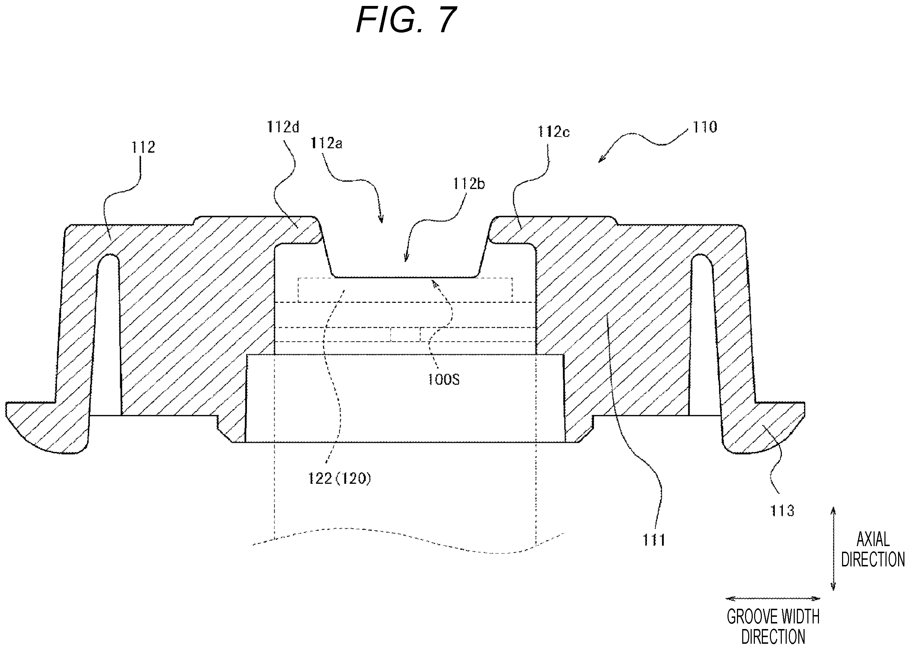

As illustrated in Fig. 4, the displacement sensor 100 is an apparatus that includes a detection surface 100S exposed to the guide groove 306a of the tube attachment unit 306 and detects a pressure applied to the detection surface 100S. The configuration of the displacement sensor 100 will be described in detail with reference to Figs. 4, 5, 6, and 7. Fig. 5 is an exploded perspective view illustrating an example of a structure of the displacement sensor 100. Fig. 6 is a perspective view of a mask member 110 of the displacement sensor 100. Fig. 7 is a cross-sectional view of the mask member 110 taken along a line I-I' in Fig. 6. As illustrated in Fig. 5, the displacement sensor 100 includes the mask member 110, a plunger 120, and an accommodating part 130.

As illustrated in Figs. 6 and 7, the mask member 110 includes a cylindrical portion 111 extending in the axial direction of the displacement sensor 100 and a flat plate portion 112 partially covering one end portion of the cylindrical portion 111. The mask member 110 is formed of, for example, plastic, but is not limited thereto. A cross-sectional shape of the cylindrical portion 111 may be arbitrarily determined depending on a shape of a distal end part 122 of the plunger 120 accommodated in an internal space of the cylindrical portion 111. In the present embodiment, the cross-sectional shape of the cylindrical portion 111 is substantially rectangular. The flat plate portion 112 defines a groove portion 112a to which a part in the extending direction of the second tube 203b is attached. As illustrated in Fig. 4, the groove portion 112a constitutes a part of the guide groove 306a in a state where the displacement sensor 100 is attached to the tube attachment unit 306.

Referring again to Fig. 6, the groove bottom of the groove portion 112a is provided with an opening portion 112b that lies in a row with the internal space of the cylindrical portion 111. Then, the flat plate portion 112 includes a first mask portion 112c that covers one end of the opening portion 112b in a groove width direction of the groove portion 112a and a second mask portion 112d that covers the other end of the opening portion 112b in the groove width direction of the groove portion 112a. The first mask portion 112c and the second mask portion 112d are separated from each other in the groove width direction of the groove portion 112a. As a result, as illustrated in Fig. 7, the distal end part 122 of the plunger 120 is inserted into the internal space of the cylindrical portion 111 of the mask member 110, so that the detection surface 100S provided at the distal end part 122 is exposed from the opening portion 112b to the groove portion 112a. Then, the first mask portion 112c covers one end of the detection surface 100S in the groove width direction of the groove portion 112a, and the second mask portion 112d covers the other end of the detection surface 100S in the groove width direction of the groove portion 112a. In the present embodiment, lengths of portions of the first mask portion 112c and the second mask portion 112d that cover the opening portion 112b in the groove width direction of the groove portion 112a are equal to each other, but may be different from each other.

As illustrated in Figs. 4 and 6, in the present embodiment, a distance between the first mask portion 112c and the second mask portion 112d in the groove width direction of the groove portion 112a is equal to a groove width of the groove portion 112a. As a result, in a case where the second tube 203b attached to the infusion pump 300 is not correctly attached to the groove portion 112a constituting a part of the guide groove 306a, the second tube 203b easily comes into contact with the first mask portion 112c or the second mask portion 112d.

However, the lengths of the first mask portion 112c and the second mask portion 112d in the groove width direction of the groove portion 112a and the distance therebetween are not limited to the above-described configuration. The first mask portion 112c and the second mask portion 112d only need to be provided in the mask member 110 so as to include a portion overlapping the detection surface 100S in the axial direction of the displacement sensor 100 in the front view of the infusion pump 300. For example, the distance between the first mask portion 112c and the second mask portion 112d in the groove width direction of the groove portion 112a may be set to 1/2 to 1/3 of a length in the groove width direction of the detection surface 100S.

As illustrated in Fig. 6, the mask member 110 includes a fitting portion 113 extending from the flat plate portion 112 in the axial direction of the displacement sensor 100. Specifically, the mask member 110 includes two fitting portions 113 extending in the axial direction of the displacement sensor 100 at both end portions of the flat plate portion 112 in the groove width direction of the groove portion 112a. As a result, as illustrated in Fig. 5, the mask member 110 is fitted into a hole portion 306b provided on a surface of the tube attachment unit 306 via the fitting portion 113 so as to cover the plunger 120 and the accommodating part 130 configured separately from the mask member 110 from the front side of the infusion pump 300. This facilitates attachment and detachment of the displacement sensor 100 to and from the tube attachment unit 306. In addition, as described above, the mask member 110 provided with the groove portion 112a, the first mask portion 112c, and the second mask portion 112d is formed separately from the plunger 120 and the accommodating part 130, and is made to be detachable from the displacement sensor 100. As a result, even in a case where the mask member 110 is contaminated by liquid leakage from the second tube 203b or the mask member 110 is damaged, only the mask member 110 is replaced, and members including relatively expensive components such as the plunger 120 and the accommodating part 130 of the displacement sensor 100 can be continuously used. However, the mask member 110 may be formed integrally with another member of the displacement sensor 100, or may be formed integrally with the main body cover 303 of the main body unit 301 of the infusion pump 300.

As illustrated in Fig. 5, the displacement sensor 100 includes a Hall element 132 and the plunger 120 that is movable with respect to the Hall element 132 and includes a magnet 124 (two magnets 124a and 124b in the present embodiment). The plunger 120 includes a base part 121, the distal end part 122, and a spring 123. In the axial direction of the displacement sensor 100, the distal end part 122 is provided at one end portion of the base part 121, and a spring 123 is provided at the other end portion. The base part 121 and the spring 123 of the plunger 120 are partially inserted into the accommodating part 130 having a box shape installed in the hole portion 306b. The distal end part 122 of the plunger 120 is inserted into the internal space of the cylindrical portion 111 of the mask member 110. On the base part 121, the two magnets 124a and 124b are arranged side by side in the axial direction of the displacement sensor 100.

The distal end part 122 includes the detection surface 100S exposed from the inside of the cylindrical portion 111 of the mask member 110 toward the front side of the infusion pump 300. As illustrated in Fig. 7, the length of the detection surface 100S in the groove width direction of the groove portion 112a is preferably longer than the groove width of the groove portion 112a constituting a part of the guide groove 306a. As a result, a large contact area can be secured between the second tube 203b attached to the guide groove 306a and the detection surface 100S, and detection accuracy can be improved of the pressure applied from the second tube 203b to the detection surface 100S. Then, since both ends of the detection surface 100S in the groove width direction of the groove portion 112a are covered by the first mask portion 112c and the second mask portion 112d, respectively, the second tube 203b is less likely to come into contact with the detection surface 100S in a state where the second tube 203b is not correctly attached to the groove portion 112a. However, the length of the detection surface 100S in the groove width direction of the groove portion 112a may be the same as the groove width of the groove portion 112a, or smaller than the groove width of the groove portion 112a.

The base part 121 and the distal end part 122 are formed of, for example, plastic, but are not limited thereto. One end of the spring 123 is attached to a protrusion 125 of the base part 121, and the other end of the spring 123 is attached to a protrusion 131 in the accommodating part 130. The spring 123 is, for example, a coil spring. The Hall element 132 is arranged on the inner surface of the accommodating part 130.

The displacement sensor 100 is configured to detect the pressure applied to the detection surface 100S on the basis of an amount of movement of the magnets 124a and 124b with respect to the Hall element 132. Specifically, when the second tube 203b is attached to the guide groove 306a of the tube attachment unit 306, in the displacement sensor 100, the distal end part 122 of the plunger 120 moves in the axial direction of the displacement sensor 100 following the pressure applied from the second tube 203b to the detection surface 100S. As a result, the magnets 124a and 124b arranged on the plunger 120 move relative to the Hall element 132, so that the Hall element 132 detects a change in magnetic flux. For that reason, the displacement sensor 100 can detect the pressure applied from the second tube 203b to the detection surface 100S on the basis of the amount of movement of the magnets 124a and 124b with respect to the Hall element 132. With such a configuration, manufacturing cost of the displacement sensor 100 can be reduced due to a simple structure. However, the displacement sensor 100 is not limited to the above-described configuration, and may be made as any apparatus capable of detecting the pressure applied to the detection surface 100S, such as a strain gauge.

The displacement sensor 100 is communicably connected to the acquisition unit 351 described later by wired communication or wireless communication. As a result, the displacement sensor 100 can transmit information such as an amount of change in magnetic flux detected by the Hall element 132 to the acquisition unit 351 as pressure information detected by the displacement sensor 100, for example.

As described above, the displacement sensor 100 includes the groove portion 112a constituting a part of the guide groove 306a to which the second tube 203b is attached and the detection surface 100S exposed to the groove portion 112a, and includes the first mask portion 112c and the second mask portion 112d that respectively cover both ends of the detection surface 100S in the groove width direction of the groove portion 112a. The first mask portion 112c and the second mask portion 112d limit the pressure applied from the second tube 203b to the detection surface 100S in a state where the second tube 203b is not correctly attached to the groove portion 112a and is in contact with the first mask portion 112c or the second mask portion 112d. For this reason, the displacement sensor 100 can function as an erroneous attachment detection sensor that detects erroneous attachment of the second tube 203b. Specifically, in a case where the second tube 203b is not correctly attached to the guide groove 306a of the tube attachment unit 306, the pressure applied from the second tube 203b to the detection surface 100S is limited, so that the pressure from the second tube 203b detected by the displacement sensor 100 decreases. As described above, it is possible to detect erroneous attachment of the second tube 203b on the basis of the pressure detected by the displacement sensor 100.

Further, the displacement sensor 100 can function as an occlusion sensor. Specifically, in a case where occlusion occurs in the second tube 203b on the downstream side from the displacement sensor 100, the liquid flowing in the portion of the second tube 203b attached to the displacement sensor 100 increases. As a result, a diameter of the second tube 203b increases, and the contact area between the second tube 203b and the detection surface 100S increases, so that the pressure from the second tube 203b detected by the displacement sensor 100 increases. On the other hand, in a case where occlusion occurs in the second tube 203b on the upstream side from the displacement sensor 100, the liquid flowing in the portion of the second tube 203b attached to the displacement sensor 100 decreases. As a result, the diameter of the second tube 203b decreases, and the contact area between the second tube 203b and the detection surface 100S decreases, so that the pressure from the second tube 203b detected by the displacement sensor 100 decreases. As described above, it is possible to detect occlusion in the second tube 203b on the basis of the pressure detected by the displacement sensor 100. In the present embodiment, one displacement sensor 100 is caused to function not only as the erroneous attachment detection sensor but also as the occlusion sensor, whereby the number of components constituting the infusion pump 300 can be reduced, and downsizing and manufacturing cost reduction of the infusion pump 300 can be achieved.

Referring again to Fig. 4, on the inner surface side of the door unit 302, a first pressing member 140a and a second pressing member 140b are provided at positions facing the upstream-side displacement sensor 100a and the downstream-side displacement sensor 100b, respectively, in a state where the door unit 302 is closed with respect to the main body unit 301. In the present embodiment, the first pressing member 140a and the second pressing member 140b are made to have the same configuration. In the following description, in a case where the first pressing member 140a and the second pressing member 140b are not particularly distinguished from each other, the pressing members are simply collectively referred to as pressing members 140.

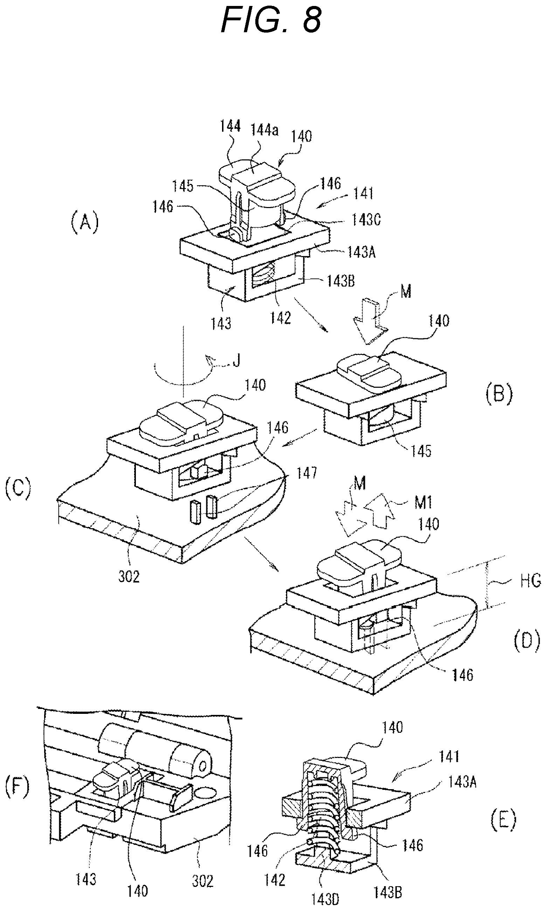

A support structure 141 of the pressing member 140 will be described with reference to Figs. 4 and 8. Fig. 8 is a perspective view illustrating an example of a structure of the pressing member 140. As illustrated in Figs. 8(A) and 8(E), the support structure 141 of the pressing member 140 includes a spring 142 as an urging member and a support member 143. The support member 143 may be formed integrally with the door unit 302. The pressing member 140 includes a pressing surface 144, a cylindrical portion 145, and two retaining portions 146. The pressing surface 144 is formed on the pressing member 140 at a position facing the displacement sensor 100 in a state where the door unit 302 is closed with respect to the main body unit 301. The two retaining portions 146 are formed at positions on left and right opposite sides of the cylindrical portion 145. One end to a middle portion of the spring 142 is accommodated in the cylindrical portion 145, and the other end of the spring 142 is fitted and held in a protrusion 143D of the support member 143. The spring 142 is, for example, a coil spring. As a result, the spring 142 functions as an urging member that urges the pressing member 140 so that the pressing surface 144 of the pressing member 140 presses the second tube 203b against the displacement sensor 100 in a state where the door unit 302 is closed with respect to the main body unit 301.

As illustrated in Fig. 8(A), the support member 143 includes a surface plate 143A and an accommodating part 143B. In the surface plate 143A, a hole portion 143C having a rectangular shape is defined that lies in a row with the accommodating part 143B having a U shape. The protrusion 143D is provided at the bottom of the accommodating part 143B. An assembly worker fits the pressing member 140 into the support member 143 by causing the cylindrical portion 145 to pass through the hole portion 143C of the support member 143. The assembly worker rotates the pressing member 140 in a J direction by 90 degrees as illustrated in Fig. 8(C) while pressing the pressing member in an M direction against force of the spring 142 as illustrated in Fig. 8(B). Then, as illustrated in Fig. 8(D), the assembly worker fits each retaining portion 146 into an engaging portion 147 provided in the door unit 302. As a result, the pressing member 140 can be pushed in the M direction against the force of the spring 142 while maintaining a state illustrated in Fig. 8(D) in the support member 143, and the pressing member 140 can be returned in the M1 direction by the force of the spring 142 in the support member 143.

As illustrated in Fig. 8, the support structure 141 of the pressing member 140 described above is adopted, whereby the pressing member 140 is easily attached to the door unit 302 by fitting the pressing member into the support member 143 and rotating the pressing member 140 by 90 degrees. For this reason, assembling workability of the pressing member 140 is improved. As illustrated in Fig. 8(D), since the support member 143 of the support structure 141 of the pressing member 140 has a simple structure and can reduce a height HG, the support member can be easily arranged on the inner surface side of the door unit 302 as illustrated in Fig. 8(F).

When the door unit 302 is closed as illustrated in Fig. 3 after the second tube 203b is attached to the tube attachment unit 306 as illustrated in Fig. 4, the first pressing member 140a and the second pressing member 140b on the door unit 302 side can press the second tube 203b against the upstream-side displacement sensor 100a and the downstream-side displacement sensor 100b, respectively. For this reason, even in a case where a plurality of types of the second tubes 203b having slight variations in outer diameter due to manufacturing tolerances or the like are attached to the infusion pump 300, the upstream-side displacement sensor 100a and the downstream-side displacement sensor 100b can more accurately detect an abnormality of the second tube 203b such as erroneous attachment or occlusion in a state where the door unit 302 is closed with respect to the main body unit 301.

Specifically, when the door unit 302 is closed, as illustrated in Fig. 5, the second tube 203b is sandwiched and held between the pressing surface 144 of the pressing member 140 and the detection surface 100S of the distal end part 122 of the plunger 120 by urging forces of the spring 123 and the spring 142. In the present embodiment, a central axis direction of the spring 123 coincides with a central axis direction of the spring 142, and the spring 123 and the spring 142 can apply pressurizing force to the second tube 203b along a diameter direction of the second tube 203b by sandwiching the second tube 203b between the pressing member 140 and the distal end part 122. For this reason, the upstream-side displacement sensor 100a or the downstream-side displacement sensor 100b can accurately detect an abnormality of the second tube 203b such as erroneous attachment or occlusion.

As illustrated in Fig. 8, the pressing surface 144 of the pressing member 140 preferably includes a protruding portion 144a. As illustrated in Fig. 4, in a state where the pressing member 140 is attached to the door unit 302, the protruding portion 144a is provided on the pressing surface 144 so as to extend along an extending direction of the guide groove 306a. A width orthogonal to an extending direction of the protruding portion 144a is made narrower than the groove width of the groove portion 112a of the displacement sensor. As a result, the protruding portion 144a is inserted into the groove portion 112a from between the first mask portion 112c and the second mask portion 112d of the displacement sensor 100 in a state where the door unit 302 is closed with respect to the main body unit 301. Since the protruding portion 144a is provided on the pressing surface 144, the pressing member 140 easily presses the second tube 203b against the displacement sensor 100 in a state where the door unit 302 is closed. For this reason, the displacement sensor 100 can more accurately detect an abnormality of the second tube 203b such as erroneous attachment or occlusion.

Preferably, the width orthogonal to the extending direction of the protruding portion 144a may be made the same as the width of the second tube 203b or slightly larger than a width of the second tube 203b depending on the second tube 203b attached to the infusion pump 300. For example, the infusion pump 300 may include a plurality of types of the pressing members 140 having different widths orthogonal to the extending direction of the protruding portion 144a. As a result, an optimum pressing member 140 may be attached to the door unit 302 depending on the second tube 203b attached to the infusion pump 300.

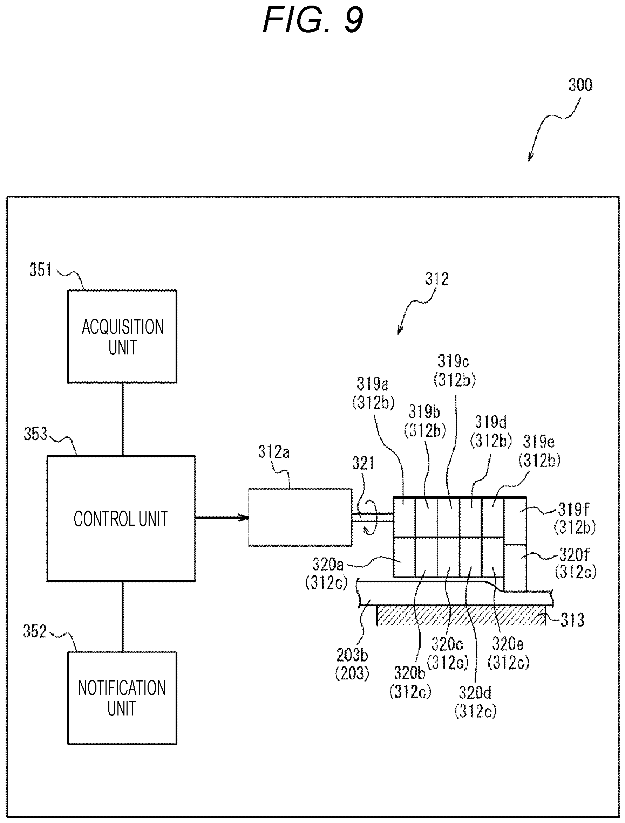

As illustrated in Fig. 9, the infusion pump 300 includes the acquisition unit 351, a notification unit 352, and a control unit 353. Fig. 9 is a block diagram of the infusion pump 300. In Fig. 9, for convenience of description, a configuration of the liquid delivery drive part 312 capable of delivering liquid in the second tube 203b is also illustrated.

The liquid delivery drive part 312 includes a drive motor 312a, a cam structure 312b including a plurality of cams rotationally driven by the drive motor 312a, and a finger structure 312c including a plurality of fingers moved by the cams of the cam structure 312b. Then, the finger structure 312c of the liquid delivery drive part 312 is arranged in the tube attachment unit 306 (see Fig. 4) described above. The cam structure 312b includes a plurality of cams, for example, six cams 319a to 319f, and the finger structure 312c includes six fingers 320a to 320f corresponding to the six cams 319a to 319f. The six cams 319a to 319f are arranged with a phase difference from each other, and the cam structure 312b is connected to an output shaft 321 of the drive motor 312a. The liquid delivery drive part 312 sequentially drives the fingers from the upstream side toward the downstream side of the second tube 203b in the extending direction of the second tube 203b. As a result, the second tube 203b is sequentially pressed and closed from the upstream side toward the downstream side of the second tube 203b, and performs peristaltic movement. For that reason, the liquid delivery drive part 312 can deliver the liquid in the second tube 203b from the upstream side toward the downstream side of the second tube 203b.

The acquisition unit 351 includes one or more communication interfaces. The acquisition unit 351 is configured to be able to acquire various types of information from other components constituting the infusion pump 300 by wired communication or wireless communication. However, the acquisition unit 351 may be configured to be able to transmit information in addition to receiving the various types of information described above. For example, the acquisition unit 351 may be able to transmit the received various types of information to an external apparatus such as a server.

For example, the acquisition unit 351 can acquire pressure information detected by the displacement sensor 100 from the displacement sensor 100 (the upstream-side displacement sensor 100a and the downstream-side displacement sensor 100b). The pressure information detected by the displacement sensor 100 is, for example, information such as the amount of change in magnetic flux detected by the Hall element 132.

In addition, for example, the acquisition unit 351 can acquire the bubble detection information from the air bubble detection sensor 307. The bubble detection information is, for example, a signal itself received by a reception unit of the air bubble detection sensor 307.

In addition, for example, the acquisition unit 351 can acquire operation mode information on the infusion pump 300. Specifically, the operation panel unit 305 (see Fig. 3 and the like) of the infusion pump 300 includes a plurality of operation buttons, and the acquisition unit 351 acquires the operation mode information depending on information input from the operation panel unit 305. More specifically, when the fast delivery switch button 305B or the start switch button 305C is pressed by an operator, the acquisition unit 351 acquires, as the operation mode information, liquid delivery mode information for executing a liquid delivery mode in which the liquid delivery drive part 312 is driven and liquid delivery is executed. In addition, when the stop switch button 305D is pressed by the operator in a state where the infusion pump 300 is in the liquid delivery mode, the acquisition unit 351 acquires, as the operation mode information, liquid delivery stop mode information for executing a liquid delivery stop mode in which the liquid delivery drive part 312 is not driven and liquid delivery is not performed.

In addition, for example, the acquisition unit 351 can acquire opened/closed state information on the door unit 302 with respect to the main body unit 301 of the infusion pump 300. Specifically, when the engaging members 314 and 315 (see Fig. 4) of the door unit 302 are fitted into the fitting portions 317 and 318 (see Fig. 4) formed in the main body unit 301, respectively, the acquisition unit 351 acquires, as the opened/closed state information, closed state information indicating that the door unit 302 is in a closed state with respect to the main body unit 301. On the other hand, when the engaging members 314 and 315 (see Fig. 4) of the door unit 302 are not in a state of being fitted into the fitting portions 317 and 318 (see Fig. 4) formed in the main body unit 301, respectively, the acquisition unit 351 acquires, as the opened/closed state information, opened state information indicating that the door unit 302 is in a state of being opened with respect to the main body unit 301.

As described above, the acquisition unit 351 can acquire information based on operation on the infusion pump 300 itself by the operator, and can acquire information transmitted from components such as the displacement sensor 100.

In addition, the acquisition unit 351 in the present embodiment acquires the pressure information from the displacement sensor 100, the bubble detection information from the air bubble detection sensor 307, the operation mode information on the infusion pump 300, and the opened/closed state information on the door unit 302 described above, but may be made to have a configuration capable of acquiring only some of the various types of information described above, or may be made to have a configuration capable of acquiring other information in addition to the various types of information described above.

The notification unit 352 includes one or more output apparatuses. The output apparatus included in the notification unit 352 is, for example, a display, a speaker, a vibrator, or the like. The notification unit 352 outputs an image, sound, vibration, or the like.

In the present embodiment, the notification unit 352 notifies the outside of the information acquired by the acquisition unit 351 as perceptual identification information that can be identified by a human on the basis of a control command from the control unit 353 described later. In the present disclosure, the "perceptual identification information" includes any information perceivable by five human senses, such as an alarm by sound as auditory identification information, and display of a color change, turning on, turning off, blinking, or the like of light of an LED or the like as visual identification information.

The control unit 353 performs instruction on operation timing or operation for each unit of the infusion pump 300, or the like. The control unit 353 includes a processor such as a CPU or an MPU. More specifically, the control unit 353 of the present embodiment includes a read only memory (ROM), a random access memory (RAM), a non-volatile memory, and a clock. The clock can correct a current time by a predetermined operation, and can execute acquisition of the current time, measurement of an elapsed time of a predetermined liquid delivery work, measurement of a reference time of speed control of liquid delivery, and the like.

In addition, the control unit 353 is connected to the power switch button 305A and a switch. The switch switches between a power converter unit and a rechargeable battery such as a lithium ion battery, to supply power to the control unit 353 from either the power converter unit or the rechargeable battery. The power converter unit is connected to a commercial AC power supply via an outlet.

In addition, the control unit 353 instructs a display unit driver to drive the display unit 304, and displays the scheduled amount of injection (mL) of administration described above, or various warning messages or the like on the display unit 304.

In addition, the control unit 353 controls the notification unit 352 on the basis of the various types of information acquired by the acquisition unit 351. For example, in a case where it is determined that an abnormality of the second tube 203b attached to the infusion pump 300 is detected on the basis of the pressure information detected by the displacement sensor 100 acquired by the acquisition unit 351, the control unit 353 controls the notification unit 352 to perform notification of the perceptual identification information. For example, the control unit 353 may display a warning message on the display unit 304 as the notification unit 352. Alternatively, the control unit 353 may cause a speaker as the notification unit 352 to output a warning sound. As a result, it is possible to urge a user of the infusion pump 300 to take measures, and improvement is achieved of safety of the infusion pump 300, and of usefulness of a technique for detecting an abnormality of the infusion tube 203 attached to the infusion pump 300.

For example, in a case where erroneous attachment of the second tube 203b is detected on the basis of the pressure detected by the displacement sensor 100, the control unit 353 outputs a warning by the notification unit 352. In a case where the pressure detected by the displacement sensor 100 is smaller than a first threshold, the control unit 353 may determine that the second tube 203b is not correctly attached to the guide groove 306a and output a warning by the notification unit 352.

In addition, for example, in a case where occlusion of the second tube 203b is detected on the basis of the pressure detected by the displacement sensor 100, the control unit 353 outputs a warning by the notification unit 352. In a case where the pressure detected by the displacement sensor 100 is smaller than a second threshold, the control unit 353 may determine that occlusion occurs in the second tube 203b on the upstream side from the displacement sensor 100 and output a warning by the notification unit 352. In a case where the pressure detected by the displacement sensor 100 is larger than a third threshold, the control unit 353 may determine that occlusion occurs in the second tube 203b on the downstream side from the displacement sensor 100 and output a warning by the notification unit 352. As described above, the infusion pump 300 detects erroneous attachment and occlusion of the second tube 203b by using one displacement sensor 100 and outputs a warning, whereby the number of components constituting the infusion pump 300 can be reduced, and downsizing and manufacturing cost reduction of the infusion pump 300 can be achieved.

In addition, the control unit 353 controls, for example, the liquid delivery drive part 312 on the basis of the various types of information acquired by the acquisition unit 351. For example, in a case where it is determined that erroneous attachment or occlusion of the second tube 203b is detected on the basis of the pressure detected by the displacement sensor 100 acquired by the acquisition unit 351, the control unit 353 may stop driving of the liquid delivery drive part 312 and end the liquid delivery mode in which liquid delivery is executed. As a result, improvement is achieved of safety of the infusion pump 300, and of usefulness of the technique for detecting an abnormality of the infusion tube 203 attached to the infusion pump 300.

Note that, even in a case where an internal pressure generated by an action of a drug delivered in the second tube 203b is constant, an amount of expansion of the second tube 203b also changes as a temperature around the second tube 203b changes. A general vinyl chloride tube or silicon tube used as the second tube 203b tends to soften as the temperature increases, and conversely, tends to harden as the temperature decreases. For this reason, even if a constant tube internal pressure acts, the second tube 203b is likely to expand when the temperature increases, and conversely, the second tube 203b is less likely to expand when the temperature decrease.

For this reason, preferably, a temperature sensor (not illustrated) such as a thermistor is provided in order to detect a use environment temperature of the infusion pump 300, and for the use environment temperature detected by the temperature sensor, an abnormality detection threshold for the second tube 203b (the first threshold, the second threshold, or the third threshold described above) is changed every 5°C within, for example, 0 to 40°C, whereby it is possible to more accurately detect an abnormality of the second tube 203b such as erroneous attachment or occlusion.

As described above, the infusion pump 300 according to the present embodiment includes the displacement sensor 100 (the upstream-side displacement sensor 100a and the downstream-side displacement sensor 100b in the present embodiment) that includes the groove portion 112a to which the infusion tube 203 (the second tube 203b in the present embodiment) is attached and the detection surface 100S exposed to the groove portion 112a, and detects the pressure applied to the detection surface 100S, in which the displacement sensor 100 includes the first mask portion 112c that covers one end of the detection surface 100S in the groove width direction of the groove portion 112a and the second mask portion 112d that covers the other end of the detection surface 100S in the groove width direction of the groove portion 112a.

With the infusion pump 300 having such a configuration, in a case where the infusion tube 203 is not correctly attached to the groove portion 112a, the pressure applied from the infusion tube 203 to the detection surface 100S is limited by the first mask portion 112c or the second mask portion 112d, so that the pressure from the infusion tube 203 detected by the displacement sensor 100 decreases. As a result, the displacement sensor 100 can detect erroneous attachment of the infusion tube 203 in the infusion pump 300 on the basis of the detected pressure. Thus, with the infusion pump 300 according to the present embodiment, it is possible to improve the usefulness of the technique for detecting an abnormality of the infusion tube 203 attached to the infusion pump 300.

Although the present disclosure has been described with reference to the drawings and examples, it should be noted that those skilled in the art can make various modifications and corrections on the basis of the present disclosure. Thus, it should be noted that these modifications and corrections fall within the scope of the present disclosure. For example, functions and the like included in means, steps, and the like can be rearranged so as not to be logically inconsistent, and a plurality of means, steps, and the like can be combined into one, or divided.

For example, in the above-described embodiment, it has been described that the infusion pump 300 is configured to be able to hold the infusion tube 203 substantially horizontally. In a case where the infusion pump 300 is configured to be able to hold the infusion tube 203 substantially horizontally, the second tube 203b is likely to be detached from the guide groove 306a of the tube attachment unit 306 due to influence of gravity, so that it is beneficial to detect erroneous attachment of the second tube 203b by the displacement sensor 100. However, a holding direction of the infusion tube 203 by the infusion pump 300 is not limited to horizontal. For example, the infusion pump 300 may be configured to be able to hold the infusion tube 203 substantially vertically. Even in a case where the infusion pump 300 is configured to be able to hold the infusion tube 203 substantially vertically, a certain effect is expected.

In addition, for example, in the above-described embodiment, it has been described that the displacement sensor 100, the acquisition unit 351, the notification unit 352, and the control unit 353 are separately arranged in the infusion pump 300. However, a configuration may be made in which the displacement sensor 100 includes the acquisition unit 351, the notification unit 352, and the control unit 353. In such a case, the displacement sensor 100 can perform a series of processing from detection of an abnormality of the infusion tube 203 to notification of a warning.

### Industrial Applicability

The present disclosure relates to an infusion pump.

### Reference Signs List

100 Displacement sensor
100a Upstream-side displacement sensor
100b Downstream-side displacement sensor
100S Detection surface
110 Mask member
111 Cylindrical portion
112 Flat plate portion
112a Groove portion
112b Opening portion
112c First mask portion
112d Second mask portion
113 Fitting portion
120 Slider
121 Base part
122 Distal end part
123 Spring
124 (124a, 124b) Magnet
125 Protrusion
130 Accommodating part
131 Protrusion
132 Hall element
140 Pressing member
140a First pressing member
140b Second pressing member
141 Support structure
142 Spring
143 Support member
143A Surface plate
143B Accommodating part
143C Hole portion
143D Protrusion
144 pressing surface
144a Protruding portion
145 Cylindrical portion
146 Retaining portion
147 Engaging portion
200 Infusion line
201 Infusion container
202 Indwelling needle
203 Tube (infusion tube)
203a First tube
203b Second tube
204 Drip tube
205 Number-of-drops abnormality detection apparatus
206 Clamp
250 Stand
300 Infusion pump
301 Main body unit
302 Door unit
303 Main body cover
304 Display unit
305 Operation panel unit
305A Power switch button
305B Fast delivery switch button
305C Start switch button
305D Stop switch button
305E Menu selection button
306 Tube attachment unit
306a Guide groove
306b Hole portion
307 Air bubble detection sensor
310 First tube guide part
311 Second tube guide part
312 Liquid delivery drive part
312a Drive motor
312b Cam structure
312c Finger structure
313 Tube pressing member
314, 315 Engaging member
316 Lever
317, 318 Fitting portion
319a to 319f Cam
320a to 320f Finger
321 Output shaft
322 Tube clamp part
351 Acquisition unit
352 Notification unit
353 Control unit
A Liquid delivery direction
I-I' Cut surface

## Claims

1. An infusion pump comprising
a displacement sensor including a groove portion to which an infusion tube is attached and a detection surface exposed to the groove portion, and detecting a pressure applied to the detection surface, wherein
the displacement sensor includes a first mask portion that covers one end of the detection surface in a groove width direction of the groove portion, and a second mask portion that covers another end of the detection surface in the groove width direction of the groove portion.

2. The infusion pump according to claim 1, wherein
the displacement sensor includes a Hall element and a plunger movable with respect to the Hall element and including a magnet, and
the displacement sensor is configured to detect a pressure applied to the detection surface on a basis of an amount of movement of the magnet with respect to the Hall element.

3. The infusion pump according to claim 1 or 2, wherein
the groove portion, the first mask portion, and the second mask portion are provided in a mask member, and
the mask member is detachable from the displacement sensor.

4. The infusion pump according to any one of claims 1 to 3, wherein a distance between the first mask portion and the second mask portion in the groove width direction of the groove portion is equal to a groove width of the groove portion.

5. The infusion pump according to any one of claims 1 to 4, comprising
a liquid delivery drive part capable of delivering liquid in the infusion tube, wherein
the displacement sensor is provided on each of both sides of the liquid delivery drive part in a liquid delivery direction of the liquid delivery drive part.

6. The infusion pump according to any one of claims 1 to 5, comprising:
a main body unit provided with the displacement sensor; and
a door unit that is openable and closable with respect to the main body unit and covers the displacement sensor in a state of being closed with respect to the main body unit, wherein
the door unit includes:
a pressing member including a pressing surface facing the displacement sensor in a state where the door unit is closed with respect to the main body unit; and
an urging member that urges the pressing member to cause the pressing surface of the pressing member to press the infusion tube against the displacement sensor in a state where the door unit is closed with respect to the main body unit.

7. The infusion pump according to claim 6, wherein
the pressing surface of the pressing member includes a protruding portion, and
the protruding portion is inserted into the groove portion from between the first mask portion and the second mask portion of the displacement sensor in a state where the door unit is closed with respect to the main body unit.

8. The infusion pump according to any one of claims 1 to 7, comprising
a notification unit, wherein
the notification unit outputs a warning in a case where erroneous attachment of the infusion tube is detected on a basis of the pressure detected by the displacement sensor.

9. The infusion pump according to claim 8, wherein the notification unit outputs a warning in a case where occlusion of the infusion tube is detected on a basis of the pressure detected by the displacement sensor.

10. The infusion pump according to any one of claims 1 to 9, wherein the infusion pump is configured to be able to hold the infusion tube substantially horizontally.
